(19) Europäisches Patentamt
European Patent Office
Office européen des brevets

(11) **EP 2 831 570 B1**

## (12) EUROPÄISCHE PATENTSCHRIFT

(45) Veröffentlichungstag und Bekanntmachung des Hinweises auf die Patenterteilung:
**15.08.2018 Patentblatt 2018/33**

(21) Anmeldenummer: **13713769.1**

(22) Anmeldetag: **18.03.2013**

(51) Int Cl.:
**G01N 21/84** (2006.01)    **G01N 21/95** (2006.01)
**G01N 33/00** (2006.01)

(86) Internationale Anmeldenummer:
**PCT/EP2013/055532**

(87) Internationale Veröffentlichungsnummer:
**WO 2013/143898 (03.10.2013 Gazette 2013/40)**

(54) **VERFAHREN ZUR DETEKTION VERGRABENER SCHICHTEN**

METHOD FOR DETECTING BURIED LAYERS

PROCÉDÉ POUR LA DÉTECTION DE COUCHES ENTERRÉES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.03.2012 DE 102012102756**

(43) Veröffentlichungstag der Anmeldung:
**04.02.2015 Patentblatt 2015/06**

(73) Patentinhaber: **Hseb Dresden GmbH**
**01099 Dresden (DE)**

(72) Erfinder:
• **SROCKA, Bernd**
**13125 Berlin (DE)**
• **LANGHANS, Ralf**
**01109 Dresden (DE)**

(74) Vertreter: **Weisse, Renate**
**Bleibtreustrasse 38**
**10623 Berlin (DE)**

(56) Entgegenhaltungen:
**US-A- 5 424 536        US-A1- 2004 223 141**
**US-A1- 2004 239 920**

## Beschreibung

**Technisches Gebiet**

Die Erfindung betrifft die Verwendung einer Anordnung, enthaltend

(a) eine Strahlungsquelle zur Beleuchtung der Oberfläche des Objekts unter Brewsterwinkel;
(b) ein im Strahlengang angeordnetes Polarisationsfilter, welches nur für Strahlung durchlässig ist, die parallel zur Einfallsebene polarisiert ist; und
(c) einen Detektor zur Erfassung von der Oberfläche des Objekts reflektierter oder von dem Objekt durchgelassener Strahlung; wobei
(d) die der Strahlungsquelle am nächsten liegenden Schichten des Objekts für die Strahlung der Strahlungsquelle zumindest teilweise durchlässig sind,

entsprechend dem Oberbegriff des Anspruchs 1.

In verschiedenen Industriezweigen werden flächige Produkte mit optischen, bildgebenden Verfahren auf ihre Eigenschaften untersucht. In der Halbleiter- und Solarzellenindustrie sind dies unter anderem Wafer. Wafer sind Scheiben aus Halbleiter-, Glas-, Folien- oder Keramikmaterialien. In verschiedenen Prozessschritten werden bei deren Herstellung in zunehmendem Maße unterschiedliche Materialien übereinander angeordnet und so Schichtstapel erzeugt.

Für die Fertigungskontrolle sowie das Qualitätsmanagement sind genaue Kenntnisse über die erzeugten Schichten erforderlich. Oft ist es jedoch aus unterschiedlichen Gründen nicht möglich, nach jedem Aufbringen einer neuen Materialschicht eine vollständige Inspektion des Werkstückes durchzuführen. In weiteren Fällen bilden sich bestimmte Schichten oder Hohlräume erst durch Prozessierung nach Aufbringen der Deckschicht aus. Sie sind daher nicht direkt zugänglich. Spezielle Analyseverfahren müssen auch in der Lage sein, vergrabene Schichten zu erreichen. Dabei ist es aus wirtschaftlichen Gründen wünschenswert, die erforderlichen Informationen mit zerstörungsfrei arbeitenden Verfahren zu generieren. Darüber hinaus müssen die Ergebnisse oft in kurzer Zeit verfügbar sein, um den Produktionsfluss durch die Inspektion nicht maßgeblich zu behindern.

**Stand der Technik**

Zur zerstörungsfreien Schichtanalyse werden zurzeit vorrangig Ellipsometrie-Messungen herangezogen. Die Eigenschaften der Schichten werden hier über die Änderung des Polarisationszustandes von eingestrahlter, polarisierter Strahlung bei der Reflexion oder Transmission an den Grenzflächen zwischen den Schichten bestimmt. Dabei wird mit einem Polarisator zunächst polarisierte Strahlung erzeugt. Mit der polarisierten Strahlung wird ein Punkt auf dem Objekt beleuchtet. Mit einem Analysator wird die Änderung des Polarisierungszustandes der reflektierten oder transmittierten Strahlung ermittelt.

Ellipsometrie-Messungen sind sehr empfindlich, arbeiten jedoch punktuell. Für eine flächenhafte Bestimmung der Schichteigenschaften müssen demzufolge eine Reihe diskreter Messpunkte aufgenommen und interpoliert werden. Der ellipsometrische Messprozess erfordert eine signifikante Messzeit je untersuchtem Punkt, da in der Regel mechanische Teile, z.B. der Analysator, bewegt werden müssen. Ellipsometrische Messungen sind daher für flächendeckende Untersuchungen mit hohen Durchsatzanforderungen nicht geeignet.

Es existieren ferner eine Reihe Verfahren der Schichtanalyse, die nicht zerstörungsfrei arbeiten. Ein Beispiel hierfür ist die Rasterelektronenmikroskopie. Die Proben müssen hierfür jedoch vertikal zu den Schichten geschnitten werden. Dadurch wird die Probe unbrauchbar.

US 5,424,536 A offenbart eine Anordnung zur Detektion von Defekten und Partikeln. Laserlicht wird hierfür unter einem Brewster-Winkel auf das Objekt gerichtet. Dies dient zur Ausschaltung des von der Oberfläche reflektierten Lichts. Durch Messung der senkrecht und der parallel polarisierten Komponente kann zwischen solchen Fehlern unterschieden werden, die auf der Oberfläche oder unterhalb der Oberfläche vorliegen.

US2004/0223141 A1 und US 2004/0239920 A1 offenbaren eine Anordnung zur Untersuchung von Waferrändern. Die bekannte Anordnung bestrahlt den Rand flächig unter Brewster-Winkel für Silizium und beobachtet die an der Oberfläche reflektierte Strahlung nach Polarisationsrichtung getrennt. Dadurch wird ein höherer Kontrast erreicht. Die bekannte Anordnung wird ausschließlich zur Untersuchung der reflektierenden Oberfläche von Waferrändern verwendet.

**Offenbarung der Erfindung**

Es ist Aufgabe der Erfindung, eine neue Verwendung für eine Anordnung der eingangs genannten Art zu schaffen. Erfindungsgemäß wird die Aufgabe dadurch gelöst, dass die Anordnung zur optischen Detektion vergrabener Schichten von flachen Objekten mit mehreren Schichten, insbesondere Wafer verwendet wird, bei denen die Medien des von den mehreren Schichten gebildeten Schichtstapels einschließlich des Einfallsmediums außer der zu detektierenden, vergrabenen Schicht lediglich zwei unterschiedliche Brechzahlen aufweisen.

Bei einer solchen Verwendung werden an geeigneten Schichtstapeln das Vorhandensein oder die Abwesenheit einer Schicht detektiert. Die Messungen erfordern nur einen geringen Aufwand und arbeiten bei hohem Durchsatz zerstörungsfrei.

Insbesondere kann die Oberfläche des Objekts mit unpolarisierter Strahlung beleuchtet werden und das Polarisationsfilter kann im Strahlengang zwischen der Oberfläche des Objekts und dem Detektor angeordnet sein.

Die Durchlässigkeit der Strahlung kann durch geeignete Auswahl des Wellenlängenbereichs erreicht werden, mit welchem das Objekt beleuchtet wird. Ein für Wafer und Halbleitersubstrate besonders geeigneter Wellenlängenbereich ist der Infrarotbereich zwischen 1 Mikrometer und 9 Mikrometer.

Der Brewsterwinkel ist materialabhängig. An der Grenzfläche von zwei Materialien mit unterschiedlichen Brechzahlen $n_1$ und $n_2$ ist für Strahlung, die aus dem Medium mit der Brechzahl $n_1$ unter dem Brewsterwinkel zum Einfallslot einfällt, der reflektierte Anteil zur Einfallsebene senkrecht polarisiert. Für den Brewsterwinkel gilt:

$$\alpha = \arctan\left(\frac{n_2}{n_1}\right)$$

Die übrige Strahlung, d.h. der gesamte parallel polarisierte Anteil der Strahlung sowie ein Teil der senkrecht polarisierten Strahlung wird in das Medium mit der Brechzahl $n_2$ übertragen.

Die reflektierte Strahlung wird durch ein Filter geleitet, das nur parallel zur Einfallsebene polarisiertes Strahlung passieren lässt. Für den soeben beschriebenen Schichtstapel würde demnach hinter dem Filter keine Strahlung registriert werden können.

In dem Medium mit der Brechzahl $n_2$ breitet sich die Strahlung nach dem Snellius'schen Brechungsgesetz in eine Richtung mit dem Winkel

$$\alpha' = \arcsin\left(\frac{n_1}{n_2}\sin\alpha\right)$$

zum Einfallslot aus. Wird nun eine weitere Schicht in dem Stapel getroffen, deren Brechzahl wieder $n_1$ ist, so trifft auch hier die Strahlung unter dem Brewsterwinkel auf die Grenzfläche. Es kann leicht gezeigt werden, dass der Brewsterwinkel für den Übergang von $n_2$ zu $n_1$ nämlich gerade $\alpha'$ ist. Als Folge wird an dieser Grenzfläche ebenfalls nur senkrecht polarisierte Strahlung reflektiert. Allgemein gilt, dass immer dann, wenn die Medien eines gesamten Schichtstapels einschließlich des Einfallsmediums lediglich zwei unterschiedliche Brechzahlen aufweisen, nur senkrecht polarisierte Strahlung reflektiert wird.

Befindet sich an irgendeiner Stelle in dem Schichtstapel ein drittes Material mit anderer Brechzahl $n_3$, die unterschiedlich zu $n_1$ und $n_2$ ist, wird die dazwischenliegende Grenzfläche zur darüber liegenden Schicht nicht unter Brewsterwinkel beleuchtet. In diesem Fall werden nun auch parallel polarisierte Anteile der Strahlung reflektiert. Die reflektierte Strahlung durchläuft den Schichtstapel in umgekehrter Folge. Sie tritt an der Oberfläche aus und kann das Filter passieren. Die durchgelassene Strahlung wird mit einem Aufnahmesystem registriert.

Für einen Schichtstapel mit den oben beschriebenen Eigenschaften kann das Verfahren sowohl für eine Positiv-Kontrolle als auch eine Negativ-Kontrolle eingesetzt werden. Bei der Positiv-Kontrolle wird bestimmt, ob eine bestimmte Schicht vollständig oder in bestimmten Gebieten vorhanden ist. Dafür werden in den überwiegend hellen Aufnahmen örtliche Helligkeitsabfälle gesucht. Bei der Negativ-Kontrolle wird bestimmt, ob eine bestimmte Schicht niemals oder nur in bestimmten Gebieten auftritt. Dabei sind auf überwiegend dunklen Aufnahmen lokale Anstiege der Helligkeit relevant.

Anders als bei der Ellipsometrie ist die flächenhafte und vollständige Abtastung der Probe möglich. Die Ergebnisse sind quasi sofort verfügbar. Die Anordnung erfordert nur wenig Aufwand und lässt sich kostengünstig einsetzen. Ferner können hohe Durchsatzraten verwirklicht werden.

Erfindungsgemäß kann der Detektor derart angeordnet sein, dass die reflektierte Strahlung erfasst wird. In einer alternativen Ausgestaltung der Erfindung kann die Anordnung auch in Transmission benutzt werden. Die Reflexion an der Schicht mit dem Brechungsindex $n_3$ schwächt die in Transmission beobachtete parallel polarisierte Strahlung, und erlaubt so die Detektion der Anwesenheit oder des Fehlens dieser Schicht. Da es hierbei nicht zu einer völligen Auslöschung der für die Detektion verwendeten Strahlung sondern nur zu einer Schwächung kommt, ist diese Modifikation vor allem geeignet, um lokale Unterschiede zu untersuchen.

In einer Ausgestaltung der Erfindung ist ein makroskopisches Aufnahmesystem vorgesehen, enthaltend eine makroskopische Beleuchtungsoptik und ein fotografisches Objektiv. Alternativ oder zusätzlich kann ein mikroskopisches Aufnahmesystem enthaltend eine mikroskopische Beleuchtungsoptik und ein mikroskopisches Objektiv vorgesehen sein.

Als Aufnahmesystem können je nach Anwendung sowohl makroskopische als auch mikroskopische Anordnungen verwendet werden. Vorteilhaft sind insbesondere Zeilendetektor-Scan-Anordnungen bei denen die Zeile horizontal über die Probe orientiert wird. Bei dieser Variante der Erfindung werden perspektivische Verzerrungen sowie Abhängigkeiten von einer optikabhängigen mitunter begrenzten Schärfentiefe eliminiert. Der Detektor kann von einem fotografischen oder elektrooptischen Empfänger, insbesondere CCD, CMOS oder InGaAs, in Form eines Zeilen- oder Flächendetektors gebildet sein, welcher eine Vielzahl von Bildpunkten gleichzeitig erfasst.

Es können auch Mittel zur Realisierung einer visuellen Beobachtung der am Objekt reflektierten oder vom Objekt transmittierten und vom Polarisationsfilter durchgelassenen Strahlung vorgesehen sein. Das kann ein Schirm oder vorzugsweise ein Okular sein.

Je nach Anwendung kann vorgesehen sein, dass eine Immersionslösung mit ausgewählter Brechzahl auf der obersten Schicht des Objekts vorgesehen ist. In vorgenannter Ausprägung der Erfindung ist die Schicht mit der Brechzahl $n_1$ Luft oder eine Immersionslösung, deren Brechzahl passend zu einer der Schichten im zu untersuchenden Stapel gewählt wurde. Dabei ist die Brechzahl also gleich $n_1$ oder $n_2$.

Hat man vor allem die Aufgabe zu lösen, lokale Unterschiede im Vorhandensein/Fehlen der Schicht mit dem Brechungsindex $n_3$ zu untersuchen, kann man auf diese Einschränkung verzichten. Dann erhält man am Übergang Luft / erste Schicht einen reflektierten Strahlungsanteil von parallel zur Einfallsebene polarisierter Strahlung. Diese Strahlungsintensität wird jedoch durch die auftretende / fehlende Reflexion an der Schicht mit $n_3$ moduliert, wodurch wiederum die Schicht mit $n_3$ geprüft werden kann. Für diese Ausführung würde man vorzugsweise eine breitbandige Strahlungsquelle einsetzen, um den Einfluss der Schichtdicken-bedingten Interferenzen zu reduzieren.

Ausgestaltungen der Erfindung sind Gegenstand der Unteransprüche. Ein Ausführungsbeispiel ist nachstehend unter Bezugnahme auf die beigefügten Zeichnungen näher erläutert.

**Kurze Beschreibung der Zeichnungen**

Fig.1 ist eine schematische Darstellung einer Messanordnung zur Untersuchung von Objekten mit mehreren Schichten.

Fig.2 illustriert Reflexion und Transmission bei Beleuchtung eines Objekts unter Brewsterwinkel mit einer Anordnung aus Figur 1.

Fig.3 zeigt einen Ausschnitt aus einem Objekt mit mehreren Schichten mit alternierenden Brechzahlen.

Fig.4 zeigt das Objekt aus Figur 3 mit einem Strahlenverlauf im Bereich einer Störung.

Fig. 5 ist eine schematische Darstellung einer am Detektor gewonnenen Aufnahme für das Objekt aus Figur 3 und 4.

**Beschreibung des Ausführungsbeispiels**

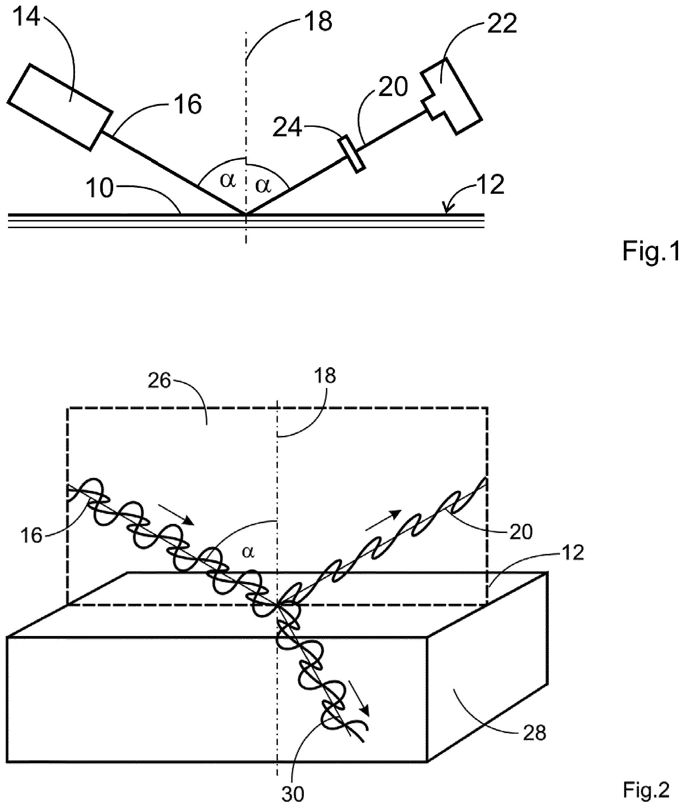

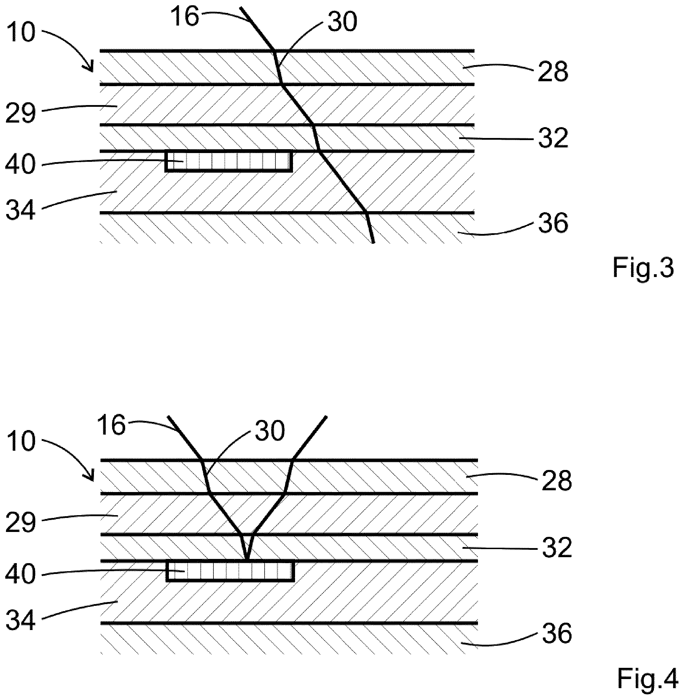

Figur 1 zeigt ein allgemein mit 10 bezeichnetes Objekt. Im vorliegenden Ausführungsbeispiel ist das Objekt 10 ein Wafer aus einem Substrat aus Silizium 28, 32 und 36 mit mehreren vertikal gestapelten luftgefüllten Kammern 29 und 34. Die Kammern 29 und 34 sind jeweils durch dünne Lagen Silizium 32 und 36 voneinander getrennt, wie dies in Figuren 3 und 4 zu erkennen ist. Herstellungsbedingt können sich in manchen der Kammern 29 und 34 Störungen in Form von Siliziumoxid-Schichten 41 ausbilden. Das entspricht der Negativ-Kontrolle.

Jedes der Materialien, d.h. Silizium, Siliziumoxid und Luft ist durch seine Brechzahl $n_x$ bestimmt. Die Brechzahl der obersten Schicht 28 aus Silizium ist 3,5. Die Brechzahl der darunterliegenden Luftschicht 29 ist 1,0. Figur 1 illustriert schematisch den Messaufbau. Die Oberfläche 12 des Objekts wird mit Infrarotstrahlung 16 aus einer Infrarot-Strahlungsquelle 14 beleuchtet. Im vorliegenden Ausführungsbeispiel wird breitbandige Strahlung des Wellenlängenbereichs zwischen 1,1 Mikrometer und 1,6 Mikrometer verwendet.

Das parallele Strahlenbündel beleuchtet eine Fläche von etwa 5x5 mm². Die Beleuchtung erfolgt unter einem Winkel von $\alpha = \arctan n_2/n_1 = \arctan 3,1/1,0 = 74°$ gegenüber der Oberflächennormalen 18. Das ist der Brewsterwinkel für den Übergang von Luft in Silizium. Eine Messung im Winkelbereich von $\pm 3°$ um den Brewsterwinkel zwischen dem Einfallsmedium und der Oberfläche 12 führt zu akzeptablen Resultaten.

Ein Teil des Strahlenbündels 16 wird reflektiert. Die reflektierte Strahlung 20 wird mit einem CCD-Flächendetektor 22 detektiert und steht zur weiteren Auswertung zur Verfügung. Im Strahlengang zwischen der Oberfläche 12 des Wafers und dem Detektor ist ein Polarisator 24 angeordnet. Der Polarisator ist so ausgerichtet, dass nur Strahlung durchgelassen wird, deren Polarisationsrichtung parallel zur Beleuchtungsebene verläuft.

Dies ist in Figur 2 illustriert. Die Beleuchtungsebene ist mit 26 bezeichnet. Sie verläuft durch die Oberflächennormale 18 und enthält sowohl die einfallende Strahlung 16 als auch die reflektierte Strahlung 20. Die Strahlung 16 ist unpolarisiert, d.h. die Verteilung der Polarisationsrichtungen ist beliebig in allen Richtungen. Da die Strahlung unter Brewsterwinkel auf das Objekt fällt, wird zunächst nur Strahlung reflektiert, deren Polarisationsrichtung senkrecht zur Beleuchtungsebene 26 ausgerichtet ist. Diese Strahlung wird am Polarisator 24 blockiert.

Die Wellenlänge der Strahlung ist so gewählt, dass das Objekt für die Strahlung im vorliegenden Ausführungsbeispiel transparent ist. Die übrige Strahlung 30 tritt daher in die oberste Schicht 28 des Objekts ein.

Figur 3 zeigt den Verlauf der Schichtstruktur des Objekts 10. Die Schichten 29 und 34 stellen die luftgefüllten Kammern dar und haben demnach dieselbe Brechzahl, wie das Medium aus dem der Lichteinfall auf das Objekt erfolgt. Die obere Schicht 28, die dazwischen liegende Schicht 32 sowie das Substrat 36 bestehen aus Silizium. Die Schicht 40 besteht aus Siliziumoxid, dessen Brechzahl weder der von Luft noch der von Silizium entspricht. Diese Schicht tritt in Objekt 10 nur an bestimmten Stellen auf und stellt somit die zu detektierende Störung dar.

Der dargestellte Strahlenverlauf in Figur 3 zeigt den parallel polarisierten Anteil. Man erkennt, dass die Strahlung an den Übergängen zwischen den Schichten 29 und 32, und zwischen den Schichten 34 und 36 unter demselben Winkel wie an der Oberfläche, nämlich dem Brewsterwinkel, einfällt. Aufgrund des oben dargestellten Zusammenhangs erfolgt auch an den Übergängen zwischen den Schichten 28 und 29 sowie 32 und 34 die Beleuchtung unter dem Brewsterwinkel. An diesen Übergängen wird lediglich Strahlung reflektiert, deren Polarisationsrichtung senkrecht zur Beleuchtungsebene 26 ausgerichtet ist. Diese Strahlung wird am Polarisator 24 blockiert. Die übrige Strahlung, insbesondere der gesamte parallel polarisierte Anteil, wird zur jeweils nächsten Schicht durchgelassen. Sofern, wie in Figur 3, dargestellt die störende Schicht 40 nicht getroffen wird, reflektiert das Objekt 10 keine parallel polarisierte Strahlung. Am Detektor 22 wird kein Signal registriert.

Figur 4 zeigt die Schichtstruktur aus Figur 3 für Strahlung, die auf die störende Schicht 40 trifft. Der Übergang zwischen den Schichten 32 und 40 wird aufgrund der abweichenden Brechzahl von Schicht 40 nicht unter dem Brewsterwinkel beleuchtet. Folglich wird hier ein Teil der Strahlung mit Polarisationsrichtung parallel zur Beleuchtungsebene reflektiert. Diese Strahlung durchläuft die Schichten in umgekehrter Folge, kann den Polarisator passieren und detektiert werden.

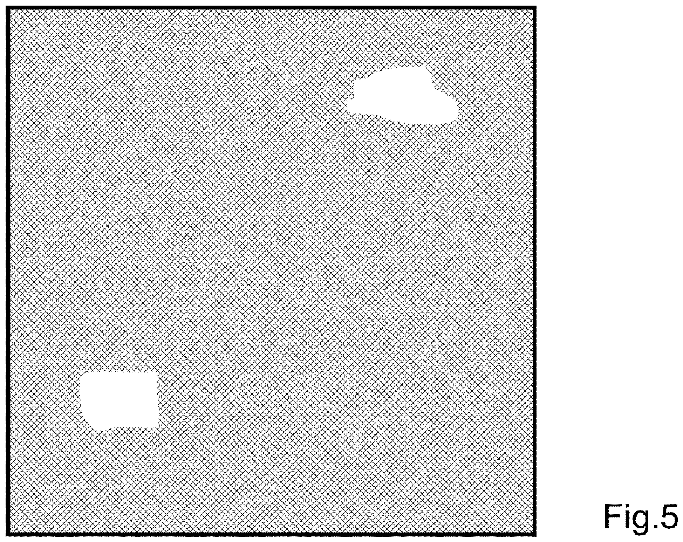

Eine typische am Detektor 22 aufgenommene Aufnahme ist schematisch in Figur 5 dargestellt. Die hellen Bereiche sind Stellen, an denen die Siliziumoxidschicht 40 vorhanden ist. Die übrigen Zonen der Aufnahme erscheinen dunkel.

Sofern der Wafer ausschließlich aus einer sich wiederholenden Schichtfolge aufgebaut ist, wird kein Signal am Detektor auftreten. Störungen durch Materialien mit einem anderen Brechungsindex führen dazu, dass an dem Übergang zu dieser Schicht nicht mehr der Brewsterwinkel getroffen wird. Umgekehrt könnte in einem anderen Fall auch das Vorhandensein einer Schicht mit anderem Brechungsindex erwünscht sein, d.h. es wird immer ein Signal am Detektor 22 detektiert. Dann wird deren Fehlen detektiert.

Die Erfindung wurde vorstehend anhand eines konkreten Ausführungsbeispiels beschrieben um deren Verständnis zu erleichtern. Es versteht sich jedoch, dass diese auch in vielfachen Variationen verwirklicht werden kann. So können Materialien mit anderen Brechungsindizes und Licht anderer Wellenlängen verwendet werden ohne dass dies einen Einfluss auf den Umfang der Erfindung hätte, der ausschließlich durch die beigefügten Ansprüche bestimmt wird.

**Patentansprüche**

1.   Verwendung einer Anordnung, enthaltend

(a) eine Strahlungsquelle (14) zur Beleuchtung der Oberfläche (12) eines Objekts (10) unter Brewsterwinkel;
(b) ein im Strahlengang angeordnetes Polarisationsfilter (24), welches nur für Strahlung (20) durchlässig ist, die parallel zur Einfallsebene (26) polarisiert ist; und
(c) einen Detektor (22) zur Erfassung von der Oberfläche (12) des Objekts (10) reflektierter (20) oder von dem Objekt (10) durchgelassener Strahlung (30); wobei
(d) die der Strahlungsquelle (14) am nächsten liegenden Schichten (28) des Objekts (10) für die Strahlung (16) der Strahlungsquelle (14) zumindest teilweise durchlässig sind;

**dadurch gekennzeichnet, dass**

(e) die Anordnung zur optischen Detektion vergrabener Schichten (40) von flachen Objekten (10) mit mehreren Schichten (28, 29, 32), insbesondere Wafer verwendet wird, bei denen die Medien des von den mehreren Schichten (28, 29, 32) gebildeten Schichtstapels einschließlich des Einfallsmediums außer der zu detektierenden, vergrabenen Schicht (40) lediglich zwei unterschiedliche Brechzahlen aufweisen.

2.   Verwendung nach Anspruch 1, **dadurch gekennzeichnet, dass** die Oberfläche (12) des Objekts (10) mit unpolarisierter Strahlung (16) beleuchtet wird und das Polarisationsfilter (24) im Strahlengang zwischen der Oberfläche (12) des Objekts (10) und dem Detektor (22) angeordnet ist.

3.   Verwendung nach einem der vorgehenden Ansprüche, **dadurch gekennzeichnet, dass** die Strahlungsquelle (14) Strahlung (16) im Infrarotbereich abstrahlt.

4.   Verwendung nach einem der vorgehenden Ansprüche, **gekennzeichnet, durch** ein makroskopisches Aufnahmesystem, enthaltend eine makroskopische Beleuchtungsoptik und ein fotografisches Objektiv.

5.   Verwendung nach einem der Ansprüche 1 bis 3, **gekennzeichnet durch** ein mikroskopisches Aufnahmesystem enthaltend eine mikroskopische Beleuchtungsoptik und ein mikroskopisches Objektiv.

6.   Verwendung nach einem der vorgehenden Ansprüche, **dadurch gekennzeichnet, dass** der Detektor (22) von einem fotografischen oder elektrooptischen Empfänger, insbesondere CCD, CMOS oder InGaAs, in Form eines Zeilen- oder Flächendetektors gebildet ist, welcher eine Vielzahl von Bildpunkten gleichzeitig erfasst.

7.   Verwendung nach einem der Ansprüche 1 bis 5, **gekennzeichnet durch** Mittel zur Realisierung einer visuellen Beobachtung der am Objekt reflektierten oder vom Objekt transmittierten und vom Polarisationsfilter (24) durchgelassenen Strahlung (20).

**8.** Verwendung nach einem der vorgehenden Ansprüche, **dadurch gekennzeichnet, dass** eine Immersionslösung mit ausgewählter Brechzahl auf der obersten Schicht (28) des Objekts (10) vorgesehen ist.

**9.** Verwendung nach einem der vorgehenden Ansprüche, **dadurch gekennzeichnet, dass** alle Schichten des Objekts (10) für die Strahlung (16) der Strahlungsquelle (14) durchlässig sind.

**Claims**

**1.** Use of an assembly comprising:

(a) a radiation source (14) for illuminating the surface (12) of an object (10) under Brewster-angle;
(b) a polarization filter (24) arranged in the path of rays which is transparent only for radiation which is polarized parallel to the plane of incidence (26); and
(c) a detector (22) for the detection of radiation reflected (20) by the surface (12) of the object (10) or transmitted (30) by the object (10); wherein
(d) the layers (28) of the object (10) which are nearest to the radiation source (14) are at least partly transparent for the radiation (16) of the radiation source (14);

**characterized in that**

(e) the assembly is used the for optical detection of buried layers (40) of flat objects (10) with a plurality of layers (28, 29, 32), in particular wafers, where the materials of the layer stack consisting of a plurality of layers (28, 29, 32) including the incident material have only two different refractive indices apart from the buried layer (40) to be detected.

**2.** Use according to claim 1, **characterized in that** the surface (12) of the object (10) is illuminated with non-polarized radiation (16) and the polarization filter (24) is positioned in the path of rays between the surface (12) of the object (10) and the detector (22).

**3.** Use according to any of the preceding claims, **characterized in that** the radiation source (14) generates radiation (16) in the infrared wavelength range.

**4.** Use according to any of the preceding claims, **characterized by** a macroscopic detection system, comprising a macroscope illumination optics and a photographic objective.

**5.** Use according to any of the claims 1 to 3, **characterized by** a microscopic detection system comprising a microscope illumination optics and a microscope objective.

**6.** Use according to any of the preceding claims, **characterized in that** the detector (22) is a photographic or electrooptical receiver, in particular a CCD, CMOS or InGaAs in the form of a line- or array detector simultaneously detecting a plurality of image points.

**7.** Use according to any of the claims 1 to 5, **characterized by** means for realizing a visual observation of the radiation (20) reflected by the object or transmitted by the object and passing the polarization filter (24).

**8.** Use according to any of the preceding claims, **characterized in that** an immersion solution having a selected refractive index is provided on top of the uppermost layer (28) of the object (10).

**9.** Use according to any of the preceding claims, **characterized in that** all layers of the object (10) are transparent for the radiation (16) from the radiation source (14).

**Revendications**

**1.** Utilisation d'une disposition comprenant

(a) une source de rayonnement (14) destinée à illuminer la surface (12) d'un objet (10) sous l'angle de Brewster ;
(b) un filtre de polarisation (24) disposé dans la marche des rayons qui ne laisse passer que le rayonnement (20) polarisé parallèlement au plan d'incidence ; et
(c) un détecteur (22) destiné à saisir le rayonnement (30) qui est réfléchi (20) par la surface (12) de l'objet (10) ou que l'objet (10) a laissé passer ;
(d) les couches (28) de l'objet (10) les plus proches de la source de rayonnement (14) laissant passer, au moins partiellement, le rayonnement (16) de la source de rayonnement (14) ;

**caractérisée en ce que**

(e) on utilise la disposition destinée à la détection optique de couches enfouies (40) d'objets plats (10) présentant plusieurs couches (28, 29, 32), notamment des tranches, pour lesquelles les milieux de la pile de couches formée de plusieurs couches (28, 29, 32), y compris le milieu d'incidence et sauf la couche enfouie (40) à détecter, présentent seulement deux indices de réfraction différents.

2. Utilisation selon la revendication 1, **caractérisée en ce que** la surface (12) de l'objet (10) est illuminée à l'aide d'un rayonnement non polarisé (16) et le filtre de polarisation (24) est disposé dans la marche des rayons entre la surface (12) de l'objet (10) et le détecteur (22).

3. Utilisation selon l'une quelconque des revendications précédentes, **caractérisée en ce que** la source de rayonnement (14) émet un rayonnement (16) situé en infrarouge.

4. Utilisation selon l'une quelconque des revendications précédentes, **caractérisée par** un système de prise de vues macroscopique comprenant un système optique d'illumination macroscopique et un objectif photographique.

5. Utilisation selon l'une quelconque des revendications 1 à 3, **caractérisée par** un système de prise de vue microscopique comprenant un dispositif optique d'illumination microscopique et un objectif microscopique.

6. Utilisation selon l'une quelconque des revendications précédentes, **caractérisée en ce que** le détecteur (22) est formé d'un récepteur photographique ou électro-optique, notamment d'un élément à couplage de charge (CCD), d'un semiconducteur d'oxyde de métal complémentaire (CMOS) ou d'un détecteur à l'arséniure d'indium-gallium (InGaAs), se présentant sous forme de détecteur lignes ou surface, qui saisit simultanément plusieurs éléments d'image.

7. Utilisation selon l'une quelconque des revendications 1 à 5, **caractérisée par** des moyens destinés à réaliser une observation visuelle du rayonnement (20) qui est réfléchi sur l'objet ou transmis par l'objet ou que le filtre de polarisation (24) a laissé passer.

8. Utilisation selon l'une quelconque des revendications précédentes, **caractérisée en ce qu'**une solution d'immersion présentant un indice de réfraction choisi est prévue sur la couche supérieure (28) de l'objet (10).

9. Utilisation selon l'une quelconque des revendications précédentes, **caractérisée en ce que** toutes les couches de l'objet (10) laissent passer le rayonnement (16) de la source de rayonnement (14).

Fig.1

Fig.2

Fig.3

Fig.4

Fig.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5424536 A
- US 20040223141 A1
- US 20040239920 A1